# EUROPEAN PATENT APPLICATION

(11) **EP 4 230 721 A1**
(43) Date of publication of application: **23.08.2023**
(21) Application number: 22157245.6
(22) Date of filing: 17.02.2022
(51) Int. Cl.: C12N 1/20, A61K 35/741, C12N 1/36, C12N 15/01

(54) **MICROORGANISMS WITH REDUCED COMPETENCE**

(71) Applicant: Evonik Operations GmbH, 45128 Essen (DE)
(72) Inventor: PELZER, Stefan, 33335 Gütersloh (DE); GRÜPPEN, Anika, 49835 Wietmarschen (DE); LENSCH, Alexandra, 64283 Darmstadt (DE); STANNEK-GÖBEL, Lorena, 33615 Bielefeld (DE); MOLCK, Stella, 33602 Bielefeld (DE); HÜSER, Andrea, 33615 Bielefeld (DE); BORGMEIER, Claudia, 64625 Bensheim (DE); DOMEYER, Jan-Eike, 64293 Darmstadt (DE); GÜNTHER, Christina, 64342 Seeheim-Jugenheim (DE)
(74) Representative: Evonik Patent Association

(57) **Abstract**

The subject of the present invention are microorganisms with reduced competence and compositions containing such microorganisms, in particular feed, food and pharmaceutical compositions.

## Description

The subject of the present invention are microorganisms with reduced competence and compositions containing such microorganisms, in particular feed, food and pharmaceutical compositions.

Many species of bacteria are able to bind and take up DNA from the surrounding medium. This capacity, which is called "competence", allows the bacterial strains to acquire new characteristics and in particular to adapt to the environment. This process is also referred to as "natural transformation" (Breitling and Dubnau (1990); J Bacteriology 172(3):1499-1508). One problem involved with this capacity, is that the strains may acquire unwanted antibiotic resistance genes which is in particular no desirable characteristic of microbial strains which are released into the environment.

Thus, the capacity of bacterial cells to acquire genetic information from the environment is a problem with respect to established production strains and in particular with respect to probiotics which are sold as feed additives and thus should not change their characteristics, neither during production nor later, when they are applied in feeding applications.

Accordingly, it was an object of the present invention to provide microbial strains with a reduced capacity to change their genetic material by acquiring genetic information from the environment due to a reduced competence.

The object of the invention could be solved by providing microorganisms with a knocked-out competence gene, wherein the knocked-out competence gene is preferably a transcription factor, more preferably the competence transcription factor *comK.*

Surprisingly, it was found out that by introducing a few point mutations into the *comK* gene the competence machinery of the microorganisms could be knocked-out, whereas the knock-out of the gene *comK* did not have any detrimental effect on the characteristics of the cells such obtained, in particular no detrimental effect regarding safety and risk aspects and also no negative influence on the probiotic activity.

Thus, a first subject of the present invention are microorganisms with reduced competence, wherein "reduced competence" preferably means that the capacity to take up DNA from the surrounding medium by natural transformation in comparison to a wild-type or parent strain is reduced by at least 50 %, more preferably by at least 60, 70 or 80 %, above all by at least 90 or 95 %. In a particularly preferred embodiment of the invention, the microorganisms of the invention are not able to take up DNA from the surrounding medium by natural transformation, at all, i.e., the competence is reduced by 100 %.

Reduction of the competence of the cells is preferably determined by stock solutions containing 25µg of plasmid DNA, more preferably as disclosed in working example 3.

The microorganisms according to the invention preferably contain at least one knocked-out competence gene or at least one competence gene which is accordingly expressed at a significantly reduced level and/or with a significantly reduced activity.

The at least one competence gene which is knocked-out or which expression or activity is significantly reduced, is preferably a gene which encodes a transcription factor, wherein very preferably the competence gene is the competence transcription factor *comK.*

Preferably the competence gene, in particular the *comK* gene, of the microorganisms of the invention comprises from 1 to 20 mutations, more preferably 1 to 10 mutations, above all 1 to 5 mutations, in comparison to a wild-type and/or parent strain, from which the microorganisms are derived, wherein the mutations are preferably point mutations. In a very preferred embodiment of the invention, at least one of said mutations, preferably at least two of said mutations, lead to the introduction of a stop codon into the sequence coding for the competence gene, in particular *comK,* wherein very preferably the *comK* parent sequence is the *comK* sequence according to SEQ ID NO: 1, as present in the *B*. *velezensis* strain CECT 5940, and wherein preferably at least one stop codon is introduced into position 1 to 50 of the sequence coding for the competence gene, in particular into the *comK* sequence. In a more preferred embodiment of the invention, the microorganism contains at least one, preferably both, of the following mutations in the *comK* gene*,* which both lead to the introduction of a stop codon, respectively: substitution of adenine by thymine at position 10; substitution of guanine by thymine at position 37. In a particularly preferred embodiment of the invention, the microorganism contains a *comK* mutant with a sequence according to SEQ ID NO: 2.

Instead and/or in addition of decreasing and/or knocking-out the activity of ComK, the activity of at least one other and/or further competence gene might be decreased and/or knocked-out, in particular by introducing at least one stop-codon into this gene. Such alternative and/or additional competence genes are in particular selected from the genes *comGA, comGB, comGC, comGD, comGE, comGF, comGG, comS, comEA, comEC, comFA, dprA, ssbB* and *recA.*

The microorganisms according to the invention can principally be obtained by any kind of method, i.e., by GMO- or non-GMO methods. But preferably the microorganisms are not genetically modified, i.e., non-GMO. This means that the microorganisms are preferably either naturally occurring microorganisms or spontaneous mutants of such naturally occurring microorganisms or microorganisms which are obtained by another method which is classified as non-GMO. The term "spontaneous mutant" refers to mutants that arise from naturally occurring microorganisms and/or parent strains without genetically modifying the microorganisms by applying classical gene technological and/or biotechnological methods like site-directed mutagenesis. Such spontaneous mutants may be obtained by classical methods of natural selection, such as growing the microorganisms in the presence of UV light and/or by applying high temperature or protoplast formation and/or in the presence of a certain antibiotic to which the parent strain is susceptible. Spontaneous mutants might be further, but less preferably, obtained by using mutagens, i.e., chemical substances which induce the formation of mutants. As formation of spontaneous mutants by using mutagens is less preferred, in a preferred embodiment of the invention the spontaneous mutants and/or non-GMO mutants are obtained without the use of such mutagens. If the mutants are obtained by applying gene technological and/or biotechnological methods like site-directed mutagenesis, then preferably methods are applied which are classified as non-GMO. A non-GMO method according to the invention is preferably characterized in that the method does not involve introduction of heterologous genetic information into the microorganism.

In a particularly preferred embodiment of the invention the microorganisms of the invention are naturally non-occurring mutants, in particular non-GMO and/or spontaneous mutants as defined before. The microorganisms of the invention have preferably the same characteristics like the parent strain from which they are derived, with exception of the reduced competence as described before. In a particularly preferred embodiment, the only difference in the genomic sequence between the microorganisms of the invention and the parent strain, from which they are derived, are said mutations in the *comK* sequence.

The microorganism of the invention may for example be obtained by the following method:
a) Introducing a reporter gene, preferably encoding a fluorescence marker, downstream to the promotor of a gene, which is activated by said regulator gene, so that activating the promotor leads to expression of the reporter gene;
b) Screening for conditions which lead to a competence induction by analyzing the microorganisms with respect to the expression level of the reporter gene;
c) Introducing a knock-out into a gene which leads to auxotrophy and at the same time to resistance with respect to a specific substance;
d) Searching for the knocked-out strains as obtained in step (c) by screening for the strains with the desired resistance;
e) Co-transformation of the strains identified in step (d) with the active form of the gene, which was knocked-out in step (c), and a knock-out variant of the regulator gene,
f) Searching for the strains which show prototrophy;
g) Screening the strains as identified in step (f) to determine whether the knock-out of the regulator gene was successful.

In this method, steps (c) and (e) and optionally also step (g) are preferably carried out at the conditions as identified in step (b) of the method.

The microorganisms of the invention are preferably obtained by the following method:
a) Introducing a reporter gene, preferably encoding a fluorescence marker, downstream to the promotor of the competence gene comGA, which is activated by ComK, so that activating the promotor leads to expression of the reporter gene,
b) Screening for conditions which lead to a competence induction by analyzing the microorganisms with respect to the expression level of the reporter gene,
c) Introducing a knock-out into a gene which leads to auxotrophy and at the same time to resistance with respect to a specific substance, wherein the gene is preferably *pyrE* and the knock-out leads preferably to resistance for 5-fluoroorotic acid (5-FOA);
d) Searching for the knocked-out strains by screening for the strains with the desired resistance, preferably resistance with respect to 5-FOA;
e) Co-transformation of the strains identified in step (d) with the active form of the gene, which was knocked-out in step (c), i.e. preferably with the active form of *pyrE,* and a knock-out variant of *comK*;
f) Searching for the strains which show prototrophy;
g) Screening the strains as identified in step (f), preferably at the conditions as identified in step (c), to determine whether the knock-out of the regulator gene *comK* was successful.

In this method, steps (c) and (e) and optionally also step (g) are preferably carried out at the conditions as identified in step (b) of the method.

The microorganisms according to the invention are preferably further non-pathogenic, in particular non-pathogenic to human beings and animals and preferably also non-pathogenic to plants.

The microorganisms according to the invention are preferably selected from a list of microorganisms generally recognized as safe ("GRAS") or listed as direct fed microbials ("DFM") or probiotics as stated in relevant listings, as for example, but not limited to the EFSA QPS list, the AAFCO list or the list by the Chinese Ministery of Agriculture (MOA).

The microorganisms according to the invention have preferably sensitivity for at least five antibiotics, more preferably for at least six, eight or ten antibiotics, above all for at least 11 or 12 antibiotics, wherein the antibiotics are preferably selected from tylosin, nalidixic acid, trimethoprim, apramycin, sulfonamide, ampicillin, vancomycin, gentamicin, kanamycin, streptomycin, erythromycin, clindamycin, tetracycline, chloramphenicol, nourseothricin, daptomycin and virginiamycin. In a very preferred embodiment the microorganisms according to the invention have sensitivity to all antibiotics as mentioned before.

To be "sensitive for antibiotics" means that growth of the microorganism is inhibited by the antibiotic under conditions where the microorganism would otherwise grow. Sensitivity for antibiotics is preferably tested in accordance with the CLSI guidelines (M07-A8 and M45-A2). A Bacillus strain is preferably considered sensitive, if growth is only detected at or below the breakpoint concentration specified in EFSA Journal 2012; 10(6):2740 for vancomycin, gentamicin, kanamycin, streptomycin, erythromycin, clindamycin, tetracycline and chloramphenicol. Concerning tylosin, nalidixic acid, trimethoprim, apramycin, sulfonamide, ampicillin, nourseothricin, daptomycin and virginiamycin, for which no breakpoint is given by EFSA for Bacillus, the breakpoint is preferably 4 mg/L.

The microorganisms according to the invention may be any kinds of microorganisms which normally possess natural competence, in particular endospore-forming bacteria and/or nonsporulating bacteria, but in a preferred embodiment of the invention, the microorganisms are endospore-forming bacteria. The cells of the endospore-forming bacteria of the invention may be present, in particular in the compositions of the invention, as spores (which are dormant), as vegetative cells (which are growing), as transition state cells (which are transitioning from growth phase to sporulation phase) or as a combination of at least two, in particular all of these types of cells. In a preferred embodiment, the compositions of the invention comprise mainly or only spores.

The microorganisms of the invention are preferably selected from endospore-forming bacteria of the phylum Bacillota (previously known as Firmicutes), preferably of the genus Bacillus, and are more preferably selected from the species *B*. *subtilis, B. amyloliquefaciens, B. velezensis, B. licheniformis, B. paralicheniformis, B. pumilus, B. megaterium, B. lentus, B. laterosporus, B. alevi, B. cereus, B. badius, B. thurigiensis, B. coagulans, B. siamensis, B. glycinifermentans, B. methylotrophicus, B. thuringensis, B. polyfermenticus, B. vallismortis, B. tequilensis, B. atrophaeus, B. mojavensis, B. sonorensis, B. inaquosus* and *B*. *safensis,* most preferably they are of the species *Bacillus velezensis* or *B*. *amyloliquefaciens.*

In a further preferred embodiment of the invention, the microorganisms of the phylum Bacillota are of the genus Clostridium, preferably of the species *C*. *butyricum, C. tyrobutyricum* or *C*. *acetobutylicum.*

In a very preferred embodiment of the invention, the microorganisms according to the invention are of the species *Bacillus velezensis,* more preferably the genomic sequence of the microorganisms is at least 99 %, in particular at least 99.5 % or 99.8 %, above all at least 99.9 % or 99.99 % identical to the genomic sequence of the *B*. *velezensis* strain CECT 5940. In a particularly preferred embodiment, the only difference in the genomic sequence between strain CECT 5940 and the strain according to the invention are said mutations in the *comK* sequence.

In a very preferred embodiment of the invention, the microorganisms according to the invention additionally possess probiotic activity and/or can be used as direct fed microbials.

The microorganisms according to the invention in particular preferably possess at least one, more preferably at least two, three, four or five, further characteristics selected from:
a) Ability to grow fast in large-scale bioreactors;
b) Ability to grow in the presence of bile, preferably in presence of 2 mM or 4 mM of bile and/or in presence of 0.3 wt.-% of bile;
c) Ability to grow in the presence of formic acid, acetic acid, propionic acid and/or lactic acid;
d) Ability to produce organic acid(s), in particular lactic acid;
e) Ability to grow under high salt conditions;
f) Ability to form endospores;
g) Ability to grow anaerobically;
h) Ability to inhibit pathogenic bacteria, in particular *C*. *perfringens, S. enteritidis, S. typhimurium, E. coli, C. difficile* and/or *Vibrio parahaemolyticus;*
i) Ability to produce at least one enzyme, preferably selected from amylase, lipase, catalase, cellulase, xylanase and protease;
j) Sensitivity with respect to selected antibiotics;
k) Ability to produce biosurfactants, in particular surfactin;
l) Ability to modulate the microbiome.

The microorganisms of the invention, in particular microorganisms of the genus Bacillus, are preferably able to grow fast in large-scale bioreactors. This means in particular heterotrophic growth in the absence of light with doubling rates lower than 60 minutes within stirred tank reactors.

The microorganisms of the invention are preferably further characterized in that they are able to grow in presence of 2 mM bile, preferably in presence of 4 mM bile, in particular characterized by an AUC5 performance value of at least 0.5, preferably at least 0.65, above all at least 0.8, and an AUC10 performance value of at least 1.2, preferably at least 1.4, above all at least 1.6, in presence of 2 mM bile and/or in that they are able to grow in presence of 0.3 wt.-% bile, in particular in presence of 0.3 wt.-% chicken bile and/or in presence of 0.3 wt.-% porcine bile, preferably characterized by being able to survive exposure to 0.3 wt.-% bile, in particular 0.3 wt.-% chicken bile and/or 0.3 wt.-% porcine bile, for at least 3 hours, preferably for at least 5 or 8 hours.

The microorganisms of the invention are preferably further characterized by being able to grow anaerobically, in particular by being able to degrade water-insoluble cellulose and/or protein under anaerobic conditions.

The microorganisms of the invention are preferably further characterized in that at least 50 %, preferably at least 70 or 90 %, of the spores survive exposure to 99°C for 20 minutes.

The microorganisms of the invention are preferably used for preparing feed or food additive compositions by mixing the microorganisms with suitable carriers.

In a particularly preferred embodiment of the invention, the microorganisms of the invention are used in the form of, preferably dried, fermentation broths, wherein "dried" preferably means a residual water content of 0.1 to 10 wt.-%, more preferably 0.2 to 5 wt.-%, above all 0.3 to 4 wt.-%.

A further subject of the present invention is therefore also a feed or food additive containing a microorganism according to the invention, wherein the microorganism may in particular be provided in form of a dried fermentation broth of such a microorganism. The feed or food additive according to the invention preferably comprises the microorganism or a dried fermentation broth thereof in an amount of 0.1 to 10 wt.-%, more preferably in an amount of 0.2 to 5 wt.-%, in particular in an amount of 0.3 to 3 wt.-%. The feed or food additive preferably further comprises a carrier, in particular in an amount of at least 80 wt.-%, preferably in an amount of at least 90 or of at least 95 wt.-%, in particular in an amount of 90 to 99.9 wt.-% or 95 to 99.8 wt.-% or 97 to 99.7 wt.-%. In a preferred embodiment of the invention the feed or food additive consists only of a mixture of the microorganism or a dried fermentation broth of such a microorganism with said carrier.

The feed or food additives preferably contain the microorganism in an amount of 1 × 10⁹ to 1 × 10¹¹ cfu (colony forming units), more preferably in an amount of 5 × 10⁹ to 5 × 10¹⁰ cfu per g feed or food additive.

The carrier as contained in the feed or food additive is preferably selected from inert formulation ingredients added to improve recovery, efficacy, or physical properties and/or to aid in packaging and administration. Such carriers may be added individually or in combination. These carriers may be selected from anti-caking agents, anti-oxidation agents, bulking agents, binders, structurants, coatings and/or protectants. Examples of useful carriers include polysaccharides (in particular starches, maltodextrins, methylcelluloses, gums, chitosan and/or inulins), protein sources (in particular skim-milk powder and/or sweet-whey powder), protein hydrolysates (in particular peptones like soy peptone and/or yeast extract), peptides, sugars (in particular lactose, trehalose, sucrose and/or dextrose), lipids (in particular lecithin, vegetable oils and/or mineral oils), salts (in particular sodium chloride, sodium carbonate, calcium carbonate, chalk, limestone, magnesium carbonate, sodium phosphate, calcium phosphate, magnesium phosphate and/or sodium citrate), and silicates (in particular clays, in particular beolite clay, amorphous silica, fumed/precipitated silicas, zeolites, Fuller's earth, Baylith^{®}, clintpolite, montmorillonite, diatomaceous earth, talc, bentonites, and/or silicate salts like aluminium, magnesium and/or calcium silicate). Suitable carriers for animal feed additives are set forth in the American Feed Control Officials, Inc.' s Official Publication, which publishes annually. See, for example Official Publication of American Feed Control Officials, Sharon Krebs, editor, 2006 edition, ISBN 1-878341-18-9. The carriers can be added after concentrating the fermentation broth containing the microorganisms of the invention and/or during and/or after drying of the fermentation broth.

The feed or food additives according to the invention preferably have a residual moisture content of 0.1 to 6 wt.-%, preferably of 0.3 to 5 wt.-%, more preferably of 0.5 to 4 wt.-%. They further preferably exhibit a water activity (a_{w} value) of 0.05 to 0.6, preferably of 0.1 to 0.5.

A further subject of the present invention is also a method of preparing the feed or food additive according to the invention, wherein the microorganisms of the invention or a dried fermentation broth of such microorganisms is mixed with a carrier, in particular as mentioned above, preferably in amount to adjust an amount of 1 × 10⁹ to 1 × 10¹¹ cfu (colony forming units), more preferably to adjust an amount of 5 × 10⁹ to 5 × 10¹⁰ cfu per g feed or food additive.

The feed additives as mentioned before, like the microorganisms itself, can be used for the preparation of feed and pharmaceutical compositions and can be added to drinking and rearing water. In a preferred embodiment 0.1 kg to 100 kg of the feed additive, in particular 1 kg to 10 kg of the feed additive, are used per ton of feed, drinking or rearing water to provide compositions which can be used for feeding animals. The feed and food compositions can be prepared by mixing the feed or food additive with typical feed or food ingredients, respectively.

A further subject of the present invention is therefore also a feed or food composition, containing a microorganism and/or a feed or food additive according to the invention and preferably at least one further feed or food ingredient.

Thus, a further subject of the present invention is also the use of a microorganism of the invention and/or of a feed or food additive of the invention for preparing a feed or food composition.

Thus, a further subject of the present invention is also a method of preparing a feed or food composition according to the invention, wherein a microorganism of the invention and/or a feed or food additive according to the invention are mixed with further feed or food additives.

A further subject of the present invention is also a pharmaceutical composition containing a microorganism according to the invention and at least on pharmaceutically acceptable carrier.

Thus, a further subject of the invention is also the use of a microorganism of the invention and/or of a feed or food additive of the invention for preparing a pharmaceutical composition, in particular a pharmaceutical composition for treating, preventing or mitigating the course of a gut disease.

Thus, a further subject of the present invention is also a method of preparing a pharmaceutical composition according to the invention, wherein a microorganism of the invention and/or a feed or food additive according to the invention are mixed with a pharmaceutically acceptable carrier.

Endospore-forming bacteria of the genus Bacillus, in particular of species *B*. *velezensis,* above all the strain *B*. *velezensis* CECT 5940, are generally known to have a beneficial effect on the gut of animals and human beings.

Thus, the microorganisms of the invention, when administered to animals, preferably enhance the health of such animals and/or improve the general physical condition of such animals and/or improve the feed conversion rate of such animals and/or decrease the mortality rate of such animals and/or increase the survival rate of such animals and/or improve the weight gain of such animals and/or increase the productivity of such animals and/or increase the disease resistance of such animals and/or modulate the immune response of such animals and/or establish or maintain a healthy gut microflora in such animals and/or improve the meat quality of such animals, in particular the meat elasticity and/or the meat hardness, and/or reduce the shedding of bacterial and/or viral pathogens through the feces of such animals. In particular, the microorganisms and compositions of the invention might be used to assist in re-establishing a healthy balance of the gut microflora after administration of antibiotics for therapeutic purposes.

A further subject of the invention is therefore a method of enhancing the health of animals and/or of improving the general physical condition of animals and/or of improving the feed conversion rate of animals and/or of decreasing the mortality rate of animals and/or of increasing the survival rates of animals and/or of improving the weight gain of animals and/or of increasing the productivity of animals and/or of increasing the disease resistance of animals and/or of modulating the immune response of animals and/or of establishing or maintaining a healthy gut microflora in animals and/or of improving the meat quality of animals and/or of reducing the shedding of bacterial and/or viral pathogens through the feces of animals, wherein at least one microorganism and/or at least one feed additive and/or at least one composition of the invention is administered to animals.

A further subject of the invention is therefore also a method of enhancing the health of human beings and/or of improving the general physical condition of human beings and/or of increasing the disease resistance of human beings and/or of modulating the immune response of human beings and/or of establishing or maintaining a healthy gut microflora in human beings, wherein at least one microorganism and/or at least one composition according to the invention is administered to human beings.

A further subject of the invention is therefore also the use of at least one microorganism and/or at least one feed additive and/or at least one composition of the invention for (preparing a composition for) enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals, wherein the at least one microorganism and/or at least one feed additive and/or at least one composition of the invention is administered to animals.

A further subject of the invention is therefore also the use of at least one microorganism and/or at least one composition of the invention for (preparing a composition for) enhancing the health of human beings and/or for improving the general physical condition of human beings and/or for increasing the disease resistance of human beings and/or for modulating the immune response of human beings and/or for establishing or maintaining a healthy gut microflora in human beings, wherein the at least one microorganism and/or at least one composition of the invention, is administered to human beings.

A further subject of the invention is therefore also at least one microorganism and/or at least one feed or food additive and/or at least one composition of the invention, for enhancing the health of animals and/or human beings and/or for improving the general physical condition of animals and/or human beings and/or for improving the feed conversion rate of animals and/or for decreasing the mortality rate of animals and/or for increasing the survival rate of animals and/or for improving the weight gain of animals and/or for increasing the productivity of animals and/or for increasing the disease resistance of animals and/or human beings and/or for modulating the immune response of animals and/or human beings and/or for establishing or maintaining a healthy gut microflora in animals and/or human beings and/or for improving the meat quality of animals and/or for reducing the shedding of bacterial and/or viral pathogens through the feces of animals.

"Increasing the productivity of animals" refers in particular to any of the following: production of more or higher quality eggs, milk or meat or increased number of offspring or improved survival of offspring.

By using the microorganisms, feed or food additives and compositions of the invention preferably an improvement of at least one of the features as mentioned before is realized, wherein realization of the feature preferably means an improvement of at least 1 %, more preferably of at least 3 or at least 5 %, in comparison to an adequate negative control. As negative control averages known in the animal husbandry field may be used, but preferably as negative control animals which are subjected to the same treatment like the animals tested are used, but without administration of the microbial preparations and/or compositions of the invention.

In particular, the microorganisms, preparations and compositions of the invention may be administered or fed to an animal in an amount effective to inhibit and/or decrease the growth of pathogenic bacteria in the animal gut. Such pathogenic bacteria include besides *Clostridia, Salmonella, Vibrio* and *Escherichia coli* in particular also *Listeria, Enterococci, Staphylococci, Aeromonas, Streptococci, Campylobacter, Shigella, Haemophilus* and *Brachyspira.* Relatedly, the methods of the present invention may be used to decrease the amount of pathogenic bacteria, viruses and protozoans shed in animal feces. The methods of the present invention may also be used to maintain or increase the growth of beneficial bacteria, such as lactic acid bacteria, in the animal gut. By decreasing pathogenic bacteria and/or increasing or maintaining beneficial bacteria, the compositions of the present invention are able to maintain an overall healthy gut microflora.

Thus, a further subject of the invention is also a method of inhibiting and/or decreasing the growth of pathogenic bacteria and/or for maintaining and/or increasing the growth of beneficial bacteria, in particular in an animal or human gut, wherein the microorganisms, preparations and/or compositions of the invention are administered to animals or humans, and wherein the pathogenic bacteria are preferably selected from *Clostridia,* in particular from *C*. *perfringens, C. difficile, C. novyi, C. septicum* and *C*. *colinum,* from *Listeria,* in particular from *L. monocytogenes, L. seeligeri* and *L. welshimeri,* from *Salmonella,* in particular *S*. *enterica* including the subspecies *enterica, arizonae, bongori* and in particular the serovars, *S*. *gallinarum, S. pullorum, S. typhimurium, S. enteritidis, S. cholerasuis, S. heidelberg, S. dublin, S. hadar, S. typhi, S. paratyphi* and *S*. *infantis,* from *Enterococci,* in particular *E*. *faecalis, E. faecium* and *E*. *cecorum,* from *Staphylococci,* in particular *S*. *aureus,* from *Aeromonas,* from *Streptococci,* in particular *S*. *suis* and *S*. *gallinaceus,* from *Campylobacter,* in particular *C*. *jejuni* and *C*. *coli,* from *Escherichia coli,* from *Haemophilus,* in particular *Haemophilus parasuis,* from *Brachyspira, in particular Brachyspira hyodysenteriae* and from *Vibrio,* in particular *V. parahaemolyticus* and *V. harveyi,* and the beneficial bacteria are preferably selected from lactic acid bacteria, in particular from lactobacilli and bifidobacteria. The pathogenic microorganisms are selected in a preferred embodiment from *Clostridia,* in particular from *C*. *perfringens, Salmonella,* in particular *S*. *enterica,* and *Vibrio,* in particular *V*. *parahaemolyticus.*

In a preferred embodiment of the invention the amount of at least one pathogenic bacterium, in particular the amount of *C*. *perfringens, S. enterica* and/or *V. parahaemolyticus,* is reduced by at least 0.5 log, more preferably by at least 1 log, 2 log, or 3 log.

Thus, a further subject of the invention are also the microorganisms, preparations and compositions of the invention for inhibiting and/or decreasing the growth of pathogenic bacteria and/or for maintaining and/or increasing the growth of beneficial bacteria, in particular in an animal or human gut, wherein the pathogenic bacteria are preferably selected from *Clostridia,* in particular from *C*. *perfringens, C. difficile, C. novyi, C. septicum* and *C*. *colinum,* from *Listeria,* in particular from *L. monocytogenes, L. seeligeri* and *L. welshimeri,* from *Salmonella,* in particular *S*. *enterica* including the subspecies *enterica, arizonae, bongori* and in particular the serovars, *S*. *gallinarum, S. pullorum, S. typhimurium, S. enteritidis, S. cholerasuis, S. heidelberg, S. dublin, S. hadar, S. typhi, S. paratyphi* and *S*. *infantis,* from *Enterococci,* in particular *E*. *faecalis, E. faecium* and E. *cecorum,* from *Staphylococci,* in particular *S*. *aureus,* from *Aeromonas,* from *Streptococci,* in particular *S*. *suis* and *S*. *gallinaceus,* from *Campylobacter,* in particular *C*. *jejuni* and *C*. *coli,* from *Escherichia coli,* from *Haemophilus,* in particular *Haemophilus parasuis,* from *Brachyspira,* in particular *Brachyspira hyodysenteriae* and from *Vibrio,* in particular *V. parahemolyticus* and *V. harveyi,* and the beneficial bacteria are preferably selected from lactic acid bacteria, in particular from lactobacilli and bifidobacteria. The pathogenic microorganisms are selected in a preferred embodiment from *Clostridia,* in particular from *C*. *difficile* and *C*. *perfringens, Salmonella,* in particular *S*. *typhimurium and S. enteritidis, Vibrio,* in particular *V. parahaemolyticus,* and *E. coli.*

The occurrence and/or increased growth of the pathogenic bacteria does or can lead to the outbreak of certain diseases. For example the occurrence and/or increased growth of *Clostridium perfringens* can lead to the outbreak of gut diseases, in particular to the outbreak of necrotic enteritis in swine and poultry. The occurrence and/or increased growth of *C. perfringens* can also lead to the outbreak of further diseases like bacterial enteritis, gangrenous dermatitis and colangiohepatitis. Even the mildest form of infection by *C. perfringens* can already be accompanied by diarrhea, which results in wet litter and by that may lead to secondary diseases like foot pad dermatitis. While *C. perfringens* type C generally is considered to be the primary cause of necrotic enteritis and necrohemorrhagic enteritis in piglets, type A has been linked to enteric disease in suckling and feeding pigs with mild necrotic enterocolitis and villous atrophy.

*Clostridium difficile* is an important emerging pathogen that causes diarrhea primarily in neonatal swine. Affected piglets may have dyspnea, abdominal distention, and scrotal edema.

*Staphylococcus aureus subsp. aureus* can cause bumblefoot in chickens, streptococcal mastitis in sows and it is capable of generating toxins that produce food poisoning in the human body.

*Salmonella* bacteria are an important cause of food poisoning of humans, which often is linked to the consumption of meat, such as poultry meat, pork or products derived therefrom. Accordingly, controlling *Salmonella* is a significant challenge for the meat-producing industry. In Europe, a baseline study conducted in 2005 on the prevalence of *Salmonella* in egg-laying flocks has shown that at the global EU-level, 20.3% of the large-scale laying hen holdings are bacteriologically positive for *S*. *enteritidis.* In some countries, the prevalence was even higher than 80% [European Food Safety Authority (2006), "Preliminary report: analysis of the baseline study on the prevalence of salmonella in laying hen flocks of Gallus gallus"*].*

*Vibrio parahaemolyticus* is in particular responsible for diseases of crustaceans like the Early Mortality Syndrome (EMS), also known as Acute Hepatopancreatic Necrosis Disease (AHPND), which affects both Giant Tiger Prawn (*Penaeus monodon*) and Whiteleg Shrimp (*Penaeus vannamei*)*.*

Pathogens can cause further diseases like polyarthritis, fibrinous polyserositis, post-weaning enteric disorders like post-weaning diarrhea and edema disease and swine dysentery.

A preferred subject of the invention is therefore a therapeutic composition for treating a disease, in particular for treating a gut disease, in particular related to bacterial infection by Clostridia, in particular by *C*. *difficile* or *C*. *perfringens,* and/or by Salmonella, in particular *S*. *enterica,* preferably *S*. *enterica subsp. enterica enteritidis,* or *S*. *typhimurium,* and/or by Vibrio, in particular *Vibrio parahaemolyticus* and/or by *E*. *coli.*

A further preferred subject in this context is therefore a therapeutic composition for treating and/or preventiing necrotic enteritis and/or necrohemorrhagic enteritis, in particular sub-clinical necrotic enteritis and/or necrohemorrhagic enteritis, in animals, preferably swine or poultry, comprising the strains and/or preparations and/or compositions of the invention.

A further preferred subject in this context is therefore a therapeutic composition for treating and/or preventing the Early Mortality Syndrome in animals, preferably animals kept in aquaculture, more preferably crustaceans, in particular shrimps and prawns, comprising the microorganisms and/or preparations and/or compositions of the invention.

A further preferred subject in this context is therefore a therapeutic composition for treating and/or preventing diseases caused by food poisoning, comprising the microorganisms and/or preparations and/or compositions of the invention.

Another preferred subject in this context is therefore a therapeutic composition for treating and/or preventing of bacterial enteritis, gangrenous dermatitis, colangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, in animals, preferably in swine or poultry, comprising the microorganisms and/or preparations and/or compositions of the invention.

A further subject of the invention is therefore also the treatment and/or prevention of a disease, in particular of a gut disease, related to bacterial infection by Clostridia, in particular *C*. *perfringens,* and/or by Salmonella, in particular *S*. *enterica,* preferably *S*. *enterica subsp. enterica enteritidis,* and/or by Vibrio, in particular *Vibrio parahaemolyticus,* wherein a microorganism and/or preparation and/or composition of the invention is administered to an animal in need thereof, wherein the animal is preferably swine or poultry.

A further subject of the invention is therefore also the treatment and/or prevention of a disease, in particular of a gut disease, preferably of necrotic enteritis or necrohemorrhagic enteritis, in particular of sub-clinical necrotic enteritis or sub-clinical necrohemorrhagic enteritis, wherein a microorganism and/or preparation and/or composition of the invention is administered to an animal in need thereof, wherein the animal is preferably swine or poultry.

A further subject of the invention is therefore also the treatment and/or prevention of a disease of aquatic animals, in particular of the Early Mortality Syndrome, wherein a microorganism and/or preparation and/or composition of the invention is administered to an animal in need thereof, wherein the animal is preferably an animal kept in aquaculture, more preferably crustaceans, in particular shrimps and prawns.

A further subject of the invention is therefore also the treatment and/or prevention of diseases caused by food poisoning, wherein a microorganism and/or preparation and/or composition of the invention is administered to an animal or human being in need thereof.

A further subject of the invention is therefore also the treatment and/or prevention of diseases caused by food poisoning, wherein a microorganism and/or preparation and/or composition of the invention is applied to an animal product, in particular to meat or eggs, to avoid the poisoning of human beings.

A further subject of the invention is therefore also the treatment and/or prevention of a disease, preferably a disease of swine or poultry, selected from bacterial enteritis, gangrenous dermatitis, colangiohepatitis, clostridiosis, diarrhea, dyspnea, abdominal distention, scrotal edema, bumblefoot, foot pad dermatitis, streptococcal mastitis, lameness, arthritis, polyarthritis, fibrinous polyserositis, post-weaning enteric disorders like post-weaning diarrhea and edema disease, dysentery, osteomyelitis, inflammation of joints and/or bone tissue, inflammation of the pericardium, splenomegaly, hepatomegaly, renomegaly, congestion, necrosis, infarction in the liver or spleen, valvular endocarditis, septicemia and/or meningitis, wherein a microorganism and/or preparation and/or composition of the invention is administered to an animal in need thereof.

The methods and uses of the microorganisms, feed or food additives and compositions of the invention can be pharmaceutical or non-pharmaceutical, in particular therapeutic or non-therapeutic. In a particularly preferred embodiment of the invention, the methods and uses are non-pharmaceutical, in particular non-therapeutic, preferably feed and food applications.

Preferred food compositions according to the invention are fermented foods and/or dairy products, in particular yoghurt, cheese, milk, butter, curd and natto. The microorganisms according to the invention may in particular be used in beverages and in milk replacers, for examples in a baby formula.

The pharmaceutical compositions according to the invention preferably contain at least one pharmaceutically acceptable carrier. They may further contain other ingredients like typical food ingredients as listed further below.

The pharmaceutical composition according to the invention is preferably a liquid, a paste, an aerosol, a tablet, a suppository or a dried composition, in particular to be administered to the pharyngeal/respiratory tract, to the gastro-intestinal tract, to the urogenital tract or to the skin. Administration might be carried out by using a medical device, for example a spray, a pump or an inhalator.

Thus, a further subject of the present invention is also a method of preparing a pharmaceutical composition, wherein microorganisms according to the invention are mixed with a pharmaceutically acceptable carrier and optionally further compounds and wherein the pharmaceutical composition may be provided in the form of a medical device.

A further subject of the present invention is in particular also a nutritional supplement containing microorganisms according to the invention and at least one pharmaceutically acceptable carrier.

In a preferred embodiment of the invention, the microorganisms and compositions of the invention are administered orally to animals or human beings. The administration is preferably carried out in form of feed and food compositions, but may also be carried out by applying the microbial preparations or feed additives of the invention to drinking or rearing water. Thus, a further subject of the present invention is water, in particular an aqueous solution or an aqueous suspension, containing at least one microorganism or feed additive according to the invention.

In particular, when they are administered to drinking or rearing water, the microorganisms and compositions of the invention may be applied in the form of compositions, in particular tablets, which after exposure to the drinking or rearing water dissolve easily and set free the microorganisms into the aqueous environment. Such compositions, in particular tablets, are a further preferred embodiment of the invention. Those compositions preferably comprise carriers like betaine salts, citrates, carbonates and/or bicarbonates like for example disclosed in WO 2020/225020.

The feed, food and pharmaceutical compositions of the invention as well as the drinking water, rearing water, effluent water or wastewater, preferably comprise microorganisms at a rate of about 1×10² to about 1×10¹⁰ CFU/g feed or ml water, preferably in an amount of about 1×10⁴ to about 1×10⁹ CFU/g feed or ml water, more preferably in an amount of 1×10⁶ to 1×10⁸ CFU/g feed or ml water.

Correspondingly, preferred amounts of feed or food additives of the invention in the feed, food and water compositions of the invention range from 0.01 wt.-% to 10 wt.-%, more preferably from 0.05 wt.-% to 5 wt.-%, in particular from 0.1 wt.-% to 2 wt.-%.

The feed, food and pharmaceutical compositions of the invention may comprise additional carriers or further typical feed ingredients or combinations thereof.

Suitable carriers for the preparation of feed, food and pharmaceutical compositions are the same as mentioned before for the preparation of the feed or food additive starting from the microorganism of the invention or a fermentation broth of such microorganisms.

Suitable typical animal feed ingredients which may be contained in the compositions according to the invention and/or used in the preparation of feed or food compositions starting from the microorganisms and/or microbial fermentation broths and/or feed or food additives according to the invention include one or more of the following: proteins, carbohydrates, fats, further probiotics, prebiotics, enzymes, vitamins, immune modulators, milk replacers, minerals, amino acids, carriers, coccidiostats, acid-based products and/or medicines, such as antibiotics.

Carbohydrates containing components which may be used according to the invention are for example forage, roughage, wheat meal, corn meal, sunflower meal or soya meal, and mixtures thereof.

Proteins containing components which may be used according to the invention are for example soya protein, pea protein, wheat gluten, corn gluten, rice, canola meal, meal of marine animals, in particular fishmeal, meal of terrestrial animals, and mixtures thereof. "Meal of marine animals" includes meat meal, meat and bone meal, blood meal, liver meal, poultry meal, silkworm meal, silkworm pupae meal and combinations thereof.

Fats are typically provided as oils of marine animals, vegetable oils or oils of microorganisms, in particular oils of microalgae, or combinations thereof. Examples of vegetable oils are soybean oil, rapeseed oil, sunflower seed oil, canola oil, cottonseed oil, flaxseed oil and palm oil. Oils of marine animals include fish oil as well as oil of krill, bivalves, squids or shrimps and further fatty oils from fish of the families Engraulidae, Carangidae, Clupeidae, Osmeridae, Scombridae and/or Ammodytidae. Examples of oils of microalgae are in particular oil of Labyrinthulea, preferably oil of Schizochytria or Thraustochytria. Besides the isolated oils the defatted biomass itself may also be used as fat source, i.e. in particular the meal of a marine animals, preferably fishmeal, or a plant meal, in particular soybean meal, rapeseed meal, sunflower meal, canola meal, cottonseed meal and/or flax seed meal.

Proteins containing components which additionally contain fats which may be used according to the invention are for example fish meal, krill meal, bivalve meal, squid meal or shrimp shells, as well as combinations thereof.

The feedstuff according to the invention has preferably a total protein content of 20 to 50 wt.-% and/or a total fat content of 1 to 15 wt.-% and/or a total carbohydrate content of 20 to 60 wt.-%.

Further probiotics (DFM) and/or microorganisms which may be used according to the invention in combination with the microorganisms and feed or food additives of the invention are preferably bacteria selected from the species *Bacillus subtilis, Bacillus licheniformis, Bacillus paralicheniformis, Bacillus lentus, Bacillus pumilus, Bacillus laterosporus, Bacillus coagulans, Bacillus alevi, Bacillus cereus, Bacillus badius, Bacillus thurigiensis, Bacillus amyloliquefaciens, Bacillus velezensis, Enterococcus faecium,* and *Pediococcus acidilactici.* Preferred examples are *Bacillus pumilus* DSM 32539 and *Bacillus subtilis* DSM 32540 (both deposited with the DSMZ on June 14, 2017 under the provisions of the Budapest Treaty on the International Recognition of the Deposit of Microorganisms for the Purpose of Patent Procedure) and derivatives thereof, *Bacillus licheniformis* DSM 32314 and *Bacillus subtilis* DSM 32315 (both deposited with the DSMZ on May 12, 2016 under the provisions of the Budapest Treaty on the International Recognition of the Deposit of Microorganisms for the Purpose of Patent Procedure) and derivatives thereof, *Bacillus subtilis* PB6 (as described in US Patent No. 7,247,299 and deposited as ATCC Accession No. PTA-6737), which is sold by Kemin under the trademark CLOSTAT^{®}, *Bacillus subtilis* C-3102 (as described in US Patent No. 4,919,936 and deposited as FERM BP- 1096 with the Fermentation Research Institute, Agency of Industrial Science and Technology, in Japan), sold by Calpis as CALSPORIN^{®}, *Bacillus subtilis* DSM 17299, as sold by Chr. Hansen under the trademark GalliPro^{®}, *Bacillus licheniformis* DSM 17236, as sold by Chr. Hansen under the trademark GalliProTect^{®}, a mixture of *Bacillus licheniformis* DSMZ 5749 and *Bacillus subtilis* DSMZ 5750 spores, as sold by Chr. Hansen under the trademark BioPlus^{®}YC, *B. subtilis* DSM 29784, as sold by Adisseo/Novozymes under the trademark Alterion^{®}, *Bacillus subtilis,* as sold by Chr. Hansen under the trademark PORCBOOST^{®}, *B. licheniformis* DSM 28710, as sold by Hevepharma under the trademark B-Act^{®}, a three-strain *Bacillus* probiotic, as sold by DuPont under the trademark Enviva^{®} PRO, the probiotic PureGro^{™}, as sold by DSM, or *Bacillus coagulans* microorganisms as described in US Patent No. 6,849,256. Other non-Bacillus probiotics, such as *Saccharomyces cerevisiae, Pichia pastoris, Aspergillus niger, Aspergillus oryzae,* or *Hansenula,* may also be used in compositions of the present invention. In particular in food compositions further probiotics which are known to be useful to the human health may be used such as lactic acid producing bacteria of the order Lactobacillales, more preferably of the families Aerococcaceae, Carnobacteriaceae, Enterococcaceae, Lactobacillaceae, Leuconotocaceae or Streptococcaceae, or of the order Bifidobacteriales, preferably of the family Bifidobacteriaceae, in particular of the genus Bifidobacterium. If said further probiotics are not formulated as part of the compositions of the present invention, they may be administered together (either at the same time or at different times) with the compositions of the present invention.

Prebiotics which may be used according to the invention are preferably oligosaccharides, in particular selected from galactooligosaccharides, sialyloligosaccharides, lactulose, lactosucrose, fructooligosaccharides, palatinose or isomaltose oligosaccharides, glycosyl sucrose, maltooligosaccharides, isomaltooligosaccharides, cyclodextrins, gentiooligosaccharides, soybean oligosaccharides, xylooligosaccharides, dextrans, pectins, polygalacturonan, rhamnogalacturonan, mannan, hemicellulose, arabinogalactan, arabinan, arabinoxylan, resistant starch, mehbiose, chitosan, agarose, inulin, tagatose, polydextrose, and alginate.

Enzymes which may be used in feed compositions according to the invention and which may aid in the digestion of feed, are preferably selected from phytases (EC 3.1 .3.8 or 3.1.3.26), xylanases (EC 3.2.1.8), galactanases (EC 3.2.1 .89), galactosidases, in particular alpha-galactosidases (EC 3.2.1.22), proteases (EC 3.4), phospholipases, in particular phospholipases A1 (EC 3.1 .1.32), A2 (EC 3.1.1.4), C (EC 3.1.4.3), and D (EC 3.1.4.4), lysophospholipases (EC 3.1 .1.5), amylases, in particular alpha-amylases (EC 3.2.1.1 ); lysozymes (EC 3.2.1 .17), glucanases, in particular betaglucanases (EC 3.2.1.4 or EC 3.2.1.6), glucoamylases, cellulases, pectinases, or any mixture thereof.

Examples of commercially available phytases include Bio-Feed^{™} Phytase (Novozymes), Ronozyme^{®} P and HiPhos^{™} (DSM Nutritional Products), Natuphos^{™} (BASF), Finase^{®} and Quantum^{®} Blue (AB Enzymes), the Phyzyme^{®} XP (Verenium/DuPont) and Axtra^{®} PHY (DuPont). Other preferred phytases include those described in e.g. WO 98/28408, WO 00/43503, and WO 03/066847.

Examples of commercially available xylanases include Ronozyme^{®} WX and G2 (DSM Nutritional Products), Econase^{®} XT and Barley (AB Vista), Xylathin^{®} (Verenium) and Axtra^{®} XB (Xylanase/beta-glucanase, DuPont). Examples of commercially available proteases include Ronozyme^{®} ProAct (DSM Nutritional Products).

Vitamins which may be used according to the invention are for example vitamin A, vitamin D3, vitamin E, vitamin K, e.g., vitamin K3, vitamin B12, biotin, choline, vitamin B1 , vitamin B2, vitamin B6, niacin, folic acid and pantothenate, e.g. Ca-D-pantothenate, or combinations thereof.

Immmune modulators which may be used are for example antibodies, cytokines, spray-dried plasma, interleukins, or interferons, or combinations thereof.

Minerals which may be used according to the invention are for example boron, cobalt, chloride, chromium, copper, fluoride, iodine, iron, manganese, molybdenum, selenium, zinc, calcium, phosphorous, magnesium, potassium, or sodium, or combinations thereof.

Amino acids which may be used according to the invention are for example lysine, alanine, threonine, methionine or tryptophan, or combinations thereof.

The compositions of the invention may further comprise betaine and/or choline and/or other physiologically effective methyl group donors. The feedstuffs may further comprise polyunsaturated fatty acids, in particular DHA and/or EPA.

Thus, a further embodiment of the invention is also a method of preparing an animal feed composition comprising mixing at least one microorganism and/or microbial preparation and/or feed additive of the invention, in particular in an amount effective to enhance animal health, in particular gut health, with feed ingredients, such as proteins, lipids and/or carbohydrates, and optionally further beneficial substances, preferably as mentioned before, to provide a feeding product. This method may comprise for example also a pelleting step.

Standard pelleting processes known to those of skill in the art may be used, including extrusion processing of dry or semi-moist feeds. Preferred pelleting temperatures are between about 65° C and about 120° C.

The microorganisms, microbial preparations, feed additives and compositions of the invention can be administered to animals in feed and/or drinking water and/or rearing water over multiple days throughout the animal's life or during particular stages or portions of the animal's life. For example, the microorganisms and/or microbial preparations and/or compositions can be administered only in a starter diet or only in a finisher diet of farm animals.

The microorganisms, microbial preparations and compositions of the invention can further be employed in a wide dosage range. Daily doses are, for example, in the range of between approximately 1 mg and approximately 500 mg, in particular in the range of approximately 5 mg and approximately 200 mg, in the range of from approximately 10 mg to approximately 100 mg or in the range of from approximately 20 to approximately 60 mg per kilogram live weight.

Preferably according to the invention always an effective amount of the microorganisms and/or microbial preparations and/or feed additives and/or compositions of the invention is used in the embodiments of the invention. The term "effective amount" refers to an amount which effects at least one beneficial effect to an animal and/or to the environment, in particular with respect to the features as already mentioned before, in comparison to an animal or environment that has not been administered the microorganisms and/or microbial preparations and/or compositions of the invention, but besides that has been administered the same diet (including feed and other compounds) or the same composition, respectively.

In case of therapeutic applications preferably a therapeutic amount of the microorganisms and/or microbial preparations and/or feed or food additives and/or compositions of the invention is used. The term "therapeutic amount" refers to an amount sufficient to ameliorate, reverse or prevent a disease state in an animal. Optimal dosage levels for various animals can easily be determined by those skilled in the art, by evaluating, among other things, the composition's ability to (i) inhibit or reduce pathogenic viruses and/or bacteria in the gastrointestinal tract, in particular in the gut, or in the oral or nasopharyngeal cavity or on the skin or in the gills at various doses, (ii) increase or maintain levels of beneficial bacteria and /or (iii) enhance animal health, in particular gut health, at various doses.

The methods of the present invention may be used for all kinds of animals, in particular all kinds of vertebrates such as mammals, aquatic animals and birds.

Animals that may benefit from the invention include but are not limited to farm animals, pets, exotic animals, zoo animals, animals used for sports, recreation or work.

In a very preferred embodiment of the invention, the animals are farm animals, which are raised for consumption or as food-producers, such as poultry, swine and ruminants.

The poultry may be selected from productive or domestic poultry, but also from fancy poultry or wild fowl. Preferred productive poultry in this context are chickens, turkeys, ducks and geese. The productive livestock in this context is preferably poultry optimized for producing young stock or poultry optimized for bearing meat. Preferred fancy poultry or wild fowl are peacocks, pheasants, partridges, chukkars, guinea fowl, quails, capercaillies, grouse, pigeons and swans, with quails being especially preferred. Further preferred poultry are ratites, in particular ostriches and emus, as well as parrots.

Ruminants according to the invention are preferably selected from cattle, goat and sheep. In one embodiment, the compositions of this invention may be fed to preruminants to enhance their health and, in particular, to decrease the incidence of diarrhea in these animals. Preruminants are ruminants, including calves, ranging in age from birth to about twelve weeks.

Pets are preferably selected from dogs, cats, domestic birds and domestic exotic animals. Animals used for sports may in particular be horses.

The aquatic animals according to the invention are preferably finfish, in particular of the class Actinopterygii, or crustaceans. Actinopterygii include, in particular, tilapia and other cichlids, carps and other cyprinids, salmons and other salmonids, catfish, in particular African catfish and pangasius, tuna, perch, cod, smelt, milkfish, gourami, seabass, in particular barramundi, seabream, grouper and snakehead fish.

Preferred types of salmon and salmonids in this context are the Atlantic salmon, red salmon (sockeye salmon), masu salmon (cherry salmon), king salmon (Chinook salmon), keta salmon (chum salmon), coho salmon, Danube salmon, Pacific salmon, pink salmon and trout. The aquatic animals according to the invention are very preferred tilapia.

Crustaceans include in particular shrimps, lobster, crabs, prawns and crayfish.

Preferred types of shrimps in this context are *Litopenaeus, Farfantepenaeus* and *Penaeus,* in particular *Penaeus stylirostris, Penaeus vannamei, Penaeus monodon, Penaeus chinensis, Penaeus occidentalis, Penaeus calif omiensis, Penaeus semisulcatus, Penaeus esculentu, Penaeus setiferus, Penaeus japonicus, Penaeus aztecus, Penaeus duorarum, Penaeus indicus,* and *Penaeus merguiensis.* Very preferred according to the invention is the shrimp *Penaeus vannamei,* also called whiteleg shrimp.

The aquatic animals, and in particular the shrimp, which are treated or fed with the microorganisms and/or preparations and/or compositions according to the invention can in particular be larvae, post-larvae or juvenile shrimp.

The aquatic animals may in particular also be fish which is subsequently processed into fish meal or fish oil. In this connection, the fish are preferably herring, pollack, cod or small pelagic fish like anchovy, blue whiting, capelin, driftfish, jack, mackerel, menhaden, sardine or scad fish. The fish meal or fish oil thus obtained, in turn, can be used in aquaculture for farming edible fish or crustaceans.

The aquatic animals may further be oysters, clams, cockles, arkshells, bivalves, mussels or scallops.

However, the aquatic animals may also be small organisms which are used as feedstuff in aquaculture. These small organisms may take the form of, for example, nematodes, small crustaceans or rotifers.

The farming of aquatic animals may take place in ponds, tanks, basins or else in segregated areas in the sea or in lakes or in rivers, in particular in this case in cages or net pens. Farming may be used for farming the finished edible fish, but also may be used for farming fry which are subsequently released so as to restock the wild fish stocks.

In salmon farming, the fish are preferably first grown into smolts in freshwater tanks or artificial watercourses and then grown on in cages or net pens which float in the sea, ponds or rivers and which are preferably anchored in bays or fjords.

Accordingly, the feed composition according to the invention is preferably a feedstuff for use in the farming of the above-mentioned animals. Preferred applications for animal feed according to the invention is in the feed of cattle, swine, poultry, fish, crustacean or companion animals, with swine, in particular weaned piglets, and poultry being particularly preferred. Spore forming microorganisms, in particular of the genus *Bacillus,* are particularly suited for this application as they produce heat resistant spores which can be mixed into feed prior to the pelleting process of animal feed. Microorganisms according to the invention are very preferably used in milk replacers for cattle, goats, sheep and swine or as post-pelleting spray-on coating for pelleted feed material.

The microorganisms and fermentation broths to be used according to the present invention can be obtained by culturing the microorganisms of the invention according to methods well known in the art, including by using the media and other methods as described for example by Elisashvili et al. (Probiotics and Antimicrobial Proteins 11, 731-747 (2019)), Ren et al. (AMB Express 8:21 (2018)) or as described in the Report 179 of the Food and Agriculture Organization of the United Nations (FAO) with the title "Probiotics in Animal Nutrition" (Bajagai et al., FAO (2016)). Conventional large-scale microbial culture processes include submerged fermentation, solid state fermentation, or liquid surface culture. Fermentation is configured to obtain high levels of colony forming units of the microbial cells. Optionally, the fermentation broth thus obtained may be washed, for example via a diafiltration process, to remove residual fermentation broth and metabolites. Fermentation broths as used for the preparation of feed or food additives according to the invention preferably contain, after the fermentation is finished, cells in an amount of 5 × 10¹⁰ to 5 × 10¹², more preferably in an amount of 1 × 10¹¹ to 4 × 10¹² cfu/g.

### Working examples

### Example 1: Searching for conditions, where uptake of external DNA is working efficiently

As parent strain was used the *B*. *velezensis* strain CECT 5940. Natural competence induction of this strain was monitored with the help of a modified fluorescent reporter strain. The modified strain encodes a genomic mNeonGreen copy (Shaner et al. (2013); Nat Methods 10(5):407-409) that is controlled by the promotor of the competence gene comGA. The reporter strain is obtained from the parent strain by applying standard cloning techniques by using the primers CACAATCACTGGCCGCAATG (SEQ ID NO: 3) and TAGACTTCCCTCCTTCCGCAC (SEQ ID NO: 4) to amplify the comGA promoter region. The homologous comGA gene transcription is induced in competent Bacillus cells by the central competence activator protein ComK.

The reporter strain was applied as spore preparation and spores were routinely activated at 80°C for 10 minutes. In order to screen for conditions enabling competence induction, activated spores were subjected to different media, applying different dilution factors and incubation times. Incubation targeting germination (1h, 25°C) was performed in Eppendorf tubes in a Thermomix, while all subsequent incubation steps after germination were performed in 96well deep well plates (DWP) in a Duetz shaking system (Kühner AG, Switzerland). After performing the sequence of incubation steps, 100µl samples were transferred from DPWs to MTPs and fluorescence was detected in a Fluostar^{®}Omega device (BMG Labtech, Germany).

It turned out that optimal fluorescence induction for the strain CECT 5940 was obtained for cells that had undergone a) germination in 100µl HS supplemented with AGFK, then subsequent incubation for 2h after addition of MG12 ad 500µl or b) germination in 200µl HS supplemented with AGFK, then subsequent incubation for 3h after addition of mF12 ad 1000µl. Longer and shorter incubation steps resulted in much lower overall fluorescence and at the same time the lack of visibly fluorescing cells in microscopic observation. In contrast, conditions with significant increase in overall fluorescence also gave rise to the detection of fluorescent cells in microscopy.

### Example 2: Generation of the safety strain

The chromosomal *comK* locus was chosen for the generation of a contained strain as it is the central competence regulator (van Sinderen et al. (1995); Mol Microbiol. 15(3):455-62). The mutagenesis targets were placed in the *comK* open reading frame. Two stop codons were introduced into *comK* via two point mutations at positions 10 and 37 and another point mutation was introduced at position 35 in order to generate a new Hindlll restriction site, that served for detection of successful mutants by colony PCR digestion. This was accomplished by using single PCR products from PCRs using primer pairs com1/com2 and com3/com4 as a template for an overlap extension PCR to create the final mutagenesis cassette. The mutations were introduced via the primers com2 and com3, which additionally share an overlapping region, suitable for the generation of the final mutagenesis cassette in the overlap extension PCR with the primer pair com1/com4.
com1 ATCATTAATACGGCCTCTATTACC (SEQ ID NO: 5)
com2 CTTTCTAAGGGTGCATGTGTTTACTGGCACATAATGTG (SEQ ID NO: 6)
com3 TGCACCCTTAGAAAGCTTCTAAGTAAATCGTTCAACC (SEQ ID NO: 7)
com4 GTGCACCGAATCAGTCCATC (SEQ ID NO: 8)

In order to select for successful introduction of DNA the chromosomal *pyrE* (encoding orotate phosphoribosyltransferase) locus was chosen as a selection marker. The gene can serve for bidirectional selection (Sakaguchi et al. (2013); Biosci Microbiota Food Health. 32(2): 59-68). Cells with inactivated *pyrE* become uracil auxotrophic and can be selected for via their resistance against 5-fluoroorotic acid (5-FOA). The *pyrE* inactivated mutagenesis cassette was designed to introduce two stop codons into the open reading frame of *pyrE* via two SNPs. This was accomplished by using single PCR products from PCRs using primer pairs pyr1/pyr2 and pyr3/pyr4 as a template for an overlap extension PCR to create the final mutagenesis cassette of 1825 bp. The mutations were introduced via the primers pyr2 and pyr3, which additionally share an overlapping region, suitable for the generation of the final mutagenesis cassette in the overlap extension PCR with the primer pair pyr1/pyr4.
pyr1 TCCGGATGGTATACGAAGTCAG (SEQ ID NO: 9)
pyr2 TCAATGTGTAATAGATGTTATGCGATAGCC (SEQ ID NO: 10)
pyr3 TCTATTACACATTGAAGCTGTCTTTTAACGCCCGAATGAG (SEQ ID NO: 11)
pyr4 TCCAGCGGAATGAAAGACAGTG (SEQ ID NO: 12)

Subsequently, for identifying the *pyrE* inactivated variants a range of 5-FOA concentrations were tested by plating the parent strain CECT 5940 on minimal medium (MM, media see Appendix) agar plates. MM plates supplemented with the solvent DMSO (used in the 5-FO stock) served as growth control. The parent strain CECT 5940 was already completely inhibited by supplementation of 10mg/L 5- FOA on MM plates. Inhibition of the parent strain with 5-FOA occurred still with the same effectivity, when agar medium was supplemented with 10 µg/ml Uracil. Subsequently, agar plates with 10mg/ml 5-FOA and 10 µg/ml Uracil concentration were successfully applied for the selection of *pyrE* inactivated mutants. The genotype of the generated *pyrE* inactivated (Uracil auxotrophic) mutant strain was confirmed by sequencing a PCR product covering the mutagenesis site.

The *pyrE* inactivated strains such obtained were used as recipient cells for a co-transformation with the mutagenesis cassette for *comK* inactivation, together with the *pyrE* wild-type cassette and subsequent selection for prototrophy. Both mutagenesis cassettes were generated by fusing two separate PCR products via overlap extension PCR. The mutagenesis site was centrally positioned with 450-500 bp of unmutated sequence serving as homologous recombination flanks. The wildtype *pyrE* cassette applied in the co-transformation along with the *comK* mutagenesis cassette was generated in a single PCR using the outer primers pyr1/pyr4 only.

Transformants that were able to grow on minimal medium (MM) agar plates were re-streaked on MM agar to verify their prototrophic status and subsequently checked for presence of the desired mutated *comK* by PCR product sequencing. Three verified clones were subsequently selected for genome sequencing (Illumina, gDNA prepared with MasterPure Grampositive gDNA Kit). The resequencing results were bioinformatically compared to the wildtype genome sequence and checked for the presence of the point mutations. All three clones displayed the desired mutations in the *comK* locus and two of them had no further mutations at non target loci. The third clone still carried mutations in *pyrE* from the progenitor strain. Based on these results the two clones without non-target mutations were selected for phenotypic analysis.

### Example 3: Evaluation of the phenotype of the comK knock-out mutants

The competence phenotype of the two verified *comK* mutated strains was evaluated by testing the transformation efficiency for plasmid DNA via natural competence. Transformation was performed using two independent cultures of each strain, using the parent strain CECT 5940 as negative control. Each transformation reaction included 25µg of one plasmid DNA stock solution (shuttle vector carrying Tet resistance) that had been isolated from an CECT 5940 transformant to maximize transformation efficiency. The parent strain reached a transformation efficiency of 0.5-1/µg DNA, whereas neither of the *comK*

## Claims

1. Microorganism with reduced competence.

2. Microorganism according to claim 1, wherein the microorganism possesses probiotic activity, in particular ability to inhibit pathogenic bacteria, preferably *C. perfringens,* ability to grow in presence of bile and/or ability to produce organic acids, preferably lactic acid.

3. Microorganism according to claim 1 or 2, wherein the competence of the microorganism is reduced by at least 20 %, more preferably by at least 50, 70 or 90 %, and wherein the microorganism is preferably not able to take up DNA by natural transformation, at all.

4. Microorganism according to any of the preceding claims, wherein the microorganism contains at least one competence gene with reduced activity, preferably at least one knocked-out competence gene, wherein the competence gene is preferably a transcription factor.

5. Microorganism according to claim 4, wherein the competence gene with reduced activity, preferably knocked-out competence gene, is *comK,* encoding the competence transcription factor ComK, and wherein the *comK* mutant gene preferably exhibits from 1 to 20, more preferably from 1 to 10, above all 1 to 5, mutations in comparison to the functionally expressed *comK* gene and wherein preferably at least one of said mutations leads to the introduction of a stop codon into the sequence of *comK.*

6. Microorganism according to any of the preceding claims, wherein the microorganism belongs to the phylum Bacillota, preferably to the genus Bacillus, more preferably to the species *B*. *subtilis, B. amyloliquefaciens, B. velezensis, B. licheniformis, B. paralicheniformis, B. pumilus, B. megaterium, B. lentus, B. laterosporus, B. alevi, B. cereus, B. badius, B. thurigiensis, B. coagulans, B. siamensis, B. glycinifermentans, B. methylotrophicus, B. thuringensis, B. polyfermenticus, B. vallismortis, B. tequilensis, B. atrophaeus, B. mojavensis, B. sonorensis, B. inaquosus* or *B*. *safensis,* in particular to the species *Bacillus subtilis, Bacillus velezensis, Bacillus amyloliquefaciens, Bacillus pumilus* or *Bacillus megaterium,* above all to the species *Bacillus amyloliquefaciens* or *Bacillus velezensis.*

7. Microorganism according to any of the preceding claims, wherein the strain is a *B*. *velezensis* strain and the genomic DNA of the strain is preferably at least 99 %, preferably at least 99.5 % or 99.9 %, above all at least 99.99 %, identical to the genomic DNA of the strain CECT 5940.

8. Method of obtaining a microorganism with reduced competence according to any one of claims 1 to 7, comprising the following steps:
a) Introducing a reporter gene, preferably encoding a fluorescence marker, downstream to the promotor of a gene, which is activated by said competence gene, so that activating the promotor leads to expression of the reporter gene;
b) Screening for conditions which lead to a competence induction by analyzing the microorganisms with respect to the expression level of the reporter gene;
c) Introducing a knock-out into a gene of the microorganism, which leads to auxotrophy of the strain and resistance of the thus transformed strain with respect to a specific substance;
d) Searching for the knocked-out strains as obtained in step (c) by screening for the strains with the desired resistance;
e) Co-transformation of the strains identified in step (d) with the active form of the gene, which was knocked-out in step (c), and a knock-out variant of the regulator gene,
f) Searching for the strains which show prototrophy;
g) Screening the strains as identified in step (f) to determine whether the knock-out of the competence gene was successful.

9. Method according to claim 8, wherein the competence gene is *comK,* the knock-out gene which is introduced in step (c) is a knock-out variant of *pyrE* and the strain becomes auxotrophic with respect to uracil and resistant with respect to 5-FOA and co-transformation in step (e) takes place with the active form of *pyrE* and a knocked-out variant of *comK.*

10. Method according to claim 8 or 9, wherein the microorganism is of the phylum Bacillota, preferably of the genus Bacillus, more preferably of the species *Bacillus subtilis, Bacillus velezensis, Bacillus amyloliquefaciens, Bacillus pumilus* or *Bacillus megaterium,* above all to the species *Bacillus amyloliquefaciens* or *Bacillus velezensis.*

11. Feed or food additive containing a microorganism according to any of claims 1 to 7.

12. Feed or food composition containing a microorganism according to any of claims 1 to 7 or a feed or food additive according to claim 11 and preferably at least one further feed or food ingredient selected from carriers, proteins, carbohydrates, fats, further probiotics, prebiotics, enzymes, vitamins, immune modulators, milk replacers, minerals, amino acids, carriers, coccidiostats, acid-based products and/or medicines, such as antibiotics.

13. Pharmaceutical composition containing a microorganism according to any of claims 1 to 7 and a pharmaceutically acceptable carrier.

14. Use of a microorganism according to any of claims 1 to 7 and/or of a feed or food additive according to claim 11 and/or of a feed or food composition according to claim 12 for preparing a composition for enhancing the health of animals and/or for improving the general physical condition of animals and/or for improving the feed conversion rate of animals and/or for decreasing the mortality rate of animals and/or for increasing the survival rates of animals and/or for improving the weight gain of animals and/or for increasing the productivity of animals and/or for increasing the disease resistance of animals and/or for modulating the immune response of animals and/or for establishing or maintaining a healthy gut microflora in animals and/or for improving the meat quality of animals and/or for reducing the shedding of bacterial and/or viral pathogens through the feces of animals.

15. Method of feeding animals, wherein the animals are fed with a microorganism according to any of claims 1 to 7 and/or with a feed additive according to claim 11 and/or with a feed composition according to claim 12.
